# EUROPÄISCHE PATENTANMELDUNG

(11) **EP 1 358 910 A1**
(43) Veröffentlichungstag der Anmeldung: **05.11.2003**
(21) Anmeldenummer: 02009882.8
(22) Anmeldetag: 02.05.2002
(51) Int. Cl.: A61P 35/00, A61K 39/00

(54) **Verfahren und Mittel zur Prävention, Hemmung und Therapie von Krebserkrankungen**

(71) Anmelder: B.R.A.H.M.S Aktiengesellschaft, 16761 Hennigsdorf (DE)
(72) Erfinder: Bergmann, Andreas, Dr., 12351 Berlin (DE)
(74) Vertreter: Andrae, Steffen, Dr.

(57) **Zusammenfassung**

Verwendungen der Ganglioside G_{M1} und/oder Asialo-G_{M1} sowie von Substanzen, die den Kohlenhydratteil dieser Ganglioside bezüglich einer Bindung an anti-G_{M1}-Antikörper und/oder anti-AG_{M1}-Antikörper simulieren, zur Herstellung von Mitteln zur Bindung oder Blockierung von anti-G_{M1}-Antikörpern und/oder anti-AG_{M1}-Antikörpern, die an natürliche Killerzellen (NKC) binden, oder zur Blockierung antigenpräsentierender Zellen und Erzeugung einer T-Zellanergie, sowie zur Herstellung eines Affinitätsmaterials zur extrakorporalen Entfernung von anti-G_{M1}-Antikörpern und/oder anti-AG_{M1}-Antikörpern, und zwar für Zwecke der Prävention, Hemmung und Therapie von malignen Krebserkrankungen.

## Beschreibung

Die vorliegende Erfindung betrifft neue therapeutische Verfahren zur Krebsprävention bzw. Krebstherapie sowie die Mittel, die in derartigen therapeutischen Verfahren verwendbar sind. Sie beruht auf dem erstmaligen Nachweis des Auftretens von Antikörpern (Autoantikörper) mit die sogenannten "natürlichen Killerzellen" (NK-Zellen) inhibierenden Eigenschaften, bei im wesentlichen allen Patienten, die an Krebserkrankungen der verschiedensten Gewebe und Organe leiden, und auf der Rolle, die diese Antikörper aufgrund ihrer NK-Zellen inhibierenden Eigenschaften im Rahmen der Entwicklung von Krebs spielen.

Begriffe wie "Neoplasma", "Krebs" oder "Karzinom" werden im Rahmen der vorliegenden Anmeldung im wesentlichen synonym für Tumorerkrankungen, insbesondere bösartige Tumorerkrankungen, verwendet. An bösartigen Neubildungen (malignen Neoplasmen), d.h. Krebs, sind in einem Land wie Deutschland pro Jahr mehr als 300.000 Männer und Frauen erkrankt, wobei die Zahl der jährlich diagnostizierten neuen Krebsfälle sowie die Sterblichkeit erheblich sind (vgl. z.B. http://www.medicine-worldwide.de/krankheiten/krebs/xxx.html, wobei /xxx zu ersetzen ist z.B. durch /allgemeines; /lungenkrebs; /brustkrebs; /prostatakrebs; /darmkrebs; /leukaemie; /corpuskarzinom;). Maligne Neoplasmen können in nahezu jedem Gewebe bzw. Organ entstehen, und je nach betroffenem Organ unterscheidet man zahlreiche Krebserkrankungen, die sich im Hinblick auf ihre statistische Häufigkeit, Prognose und Therapierbarkeit erheblich voneinander unterscheiden können.

Es ist in diesem Zusammenhang bekannt, dass für bestimmte Personen mit einer besonderen Disposition zur Entwicklung von Krebserkrankungen, ggf. in Verbindung mit einer beruflichen Exposition, einer Medikation oder einer Vorerkrankung, die mit hoher statistischer Häufigkeit bösartig entartet, d.h. sich in einen Krebs verwandelt, ein erhöhtes Krebsrisiko besteht. Solche besonders gefährdete Personen zu erkennen und diese dann durch eine verstärkte Überwachung und/oder geeignete präventive Maßnahmen schützen zu können, ist ein wichtiges Ziel der modernen Krebsforschung. Ein in diese Richtung zielender Forschungsansatz betrifft die Genomforschung. Ein anderer, immunologischer Ansatz zur Erkennung eines besonderen Krebsrisikos und zur sich daraus ergebenden Krebsprävention, Krebshemmung und Krebstherapie ist Gegenstand der vorliegenden Anmeldung.

Zu den oben erwähnten Vorerkrankungen, für die bekannt ist, dass sie in ihrem späteren Verlauf mit einer hohen Wahrscheinlichkeit zu malignen Erkrankungen (Darmkrebs) entarten, gehören beispielsweise die sogenannten chronisch entzündlichen Darmerkrankungen, die unter den Bezeichnungen Morbus Crohn (Synonyma: Enteritis regionalis, Crohn Krankheit) und Colitis ulcerosa (Synonyma: Colitis gravis) sowie Colitis indeterminata, einer Mischform der beiden vorgenannten Erkrankungen, bekannt sind. Diese sind aufgrund der hohen assoziierten Morbidität (Erkrankungsrate) und Mortalität (Sterberate) von besonderer Bedeutung für die Volkswirtschaft und die innere Medizin. Charakteristische Symptome sind abdominelle Schmerzen, Diarrhöen, und bei Dünndarmbefall ggf. Malabsorption. Als Komplikationen treten beim Morbus Crohn Darmstenosen, innere und äußere Fisteln und Abszessbildungen auf, bei der Colitis ulcerosa dagegen zum Teil schwere Blutverluste und ein signifikant erhöhtes Risiko des Auftretens von Dickdarmkarzinomen (ca. 40% bei 25-jährigem Krankheitsverlauf). Wäre es z.B. möglich, innerhalb der Gruppe der an chronisch entzündlichen Darmerkrankungen leidenden Patienten besonders gefährdete Personen zu erkennen und diese therapeutisch verstärkt zu schützen, wäre das ein erheblicher medizinscher Fortschritt.

In den letzten Jahren wurde eine Vielzahl von Therapien entwickelt, die im Hinblick auf die Heilbarkeit zahlreicher Formen von Krebserkrankungen erhebliche Fortschritte mit sich gebracht haben. Ganz besonders wichtig ist dabei jedoch in allen Fällen eine Früherkennung von Krebs, d.h. eine Erkennung von Neoplasmen, zu einem Zeitpunkt, zu dem noch keine oder noch keine signifikanten klinischen Symptome auftreten.

Zur möglichst frühen Erkennung von Neoplasmen in der klinischen Diagnose werden in biologischen Proben, insbesondere Blutproben, von untersuchten Patienten sog. "Tumormarker" bestimmt. Tumormarker sind Substanzen, die entweder von malignen Tumorzellen direkt gebildet werden oder die dadurch entstehen, dass Tumorzellen die Synthese des jeweiligen Markers in Nicht-Tumorzellen induzieren. Werden Tumormarker in erhöhter Konzentration in flüssigen biologischen Proben (humorale Tumormarker) bzw. im Gewebe lokalisiert (zelluläre Tumormarker), ermöglichen sie Schlüsse auf das Vorliegen, den Verlauf und die Prognose einer Tumorerkrankung. Die derzeit in der klinischen diagnostischen Praxis eingeführten Tumormarker können onkofetale Antigene, mit monoklonalen Antikörpern erkennbare Kohlenhydratepitope, Enzyme, Isoenzyme, onkogene Produkte und Rezeptoren sein. Ein Überblick über die derzeit in der klinischen Diagnostik genutzten Tumormarker findet sich in z.B. in: Lothar Thomas (Herausgeber), Labor und Diagnose, 5. erw. Auflage, Kapitel 34: Tumormarker, S. 956-1019;

Trotz der angesprochenen Fortschritte der Krebstherapie besteht weiterhin ein großer Bedarf nach neuen verbesserten Krebstherapien und Krebs-Präventionsmaßnahmen bzw. neuen im genannten Sinne wirksamen Mitteln, insbesondere solchen, die an einem zentralen Punkt der Krebsentstehung und Entwicklung ansetzen.

Die vorliegende Erfindung beruht auf der überraschenden Feststellung, dass ein bestimmter, an sich aus anderen Zusammenhängen bekannter Antikörper- bzw. Autoantikörper-Typ, der NK-Zellen inhibieende Eigenschaften aufweist, soweit derzeit experimentell überprüfbar war, bei allen getesteten malignen Neoplasmen (Krebsarten) in biologischen Proben, insbesondere Patientenseren, diagnostisch signifikant erhöht gefunden wird, während der gleiche Antikörper bei gesunden Normalpersonen nicht oder nur in deutlich geringeren Mengen nachweisbar ist. Aufgrund der NK-Zellen inhibierenden Eigenschaften ist die dieser Zelltyp ursächlich mit einem Krebsgeschehen verknüpft.

Indem man diesen Antikörper bestimmt, wird es gemäß der vorliegenden Erfindung möglich, z.B. bei Patienten oder Personen, die sich einer Routineuntersuchung unterziehen oder an einer Reihenuntersuchung teilnehmen, mit hoher Zuverlässigkeit das Vorliegen maligner Neoplasmen zu erkennen, ohne dass eine signifikante Anzahl von gesunden Normalpersonen falsch positiv ermittelt wird. Soweit der bestimmte Antikörper auch bei einigen speziellen anderen Erkrankungen in erhöhten Konzentrationen auftritt, ist in der Regel eine korrekte Interpretation der Meßergebnisse unter Ausschluß eienr solchen Erkrankung aufgrund zusätzlicher klinischer Befunde ohne größere Schwierigkeiten möglich.

Werden die NK-Zellen inhibierenden Antikörper bei einem Patienten nachgewiesen, werden sie zu einem therapeutisches Target. Sie können dann unter Anwendung von z.T. an sich grundsätzlich bekannten Maßnahmen bzw. erfindungsgemäßen Mitteln zur Bekämpfung, Bindung und Unschädlichmachung pathogener (Auto-)Antikörper und/oder von Maßnahmen mit dem Zile einer gezielten Beeinflussung der Antikörperbildung durch das Immunsystem zum Ziel präventiver, hemmender und/oder therapeutischer Maßnahmen werden.

Die vorliegende Erfindung geht aus von den überraschenden Befunden von Vermessungen von Seren von Normalpersonen und Krebspatienten durch die Anmelderin mit Hilfe eines Ligandenbindungsassays mit erhöhter Sensitivität für an die Ganglioside AG_{M1} und G_{M1} bindende Antikörper, nämlich dass im wesentlichen in allen vermessenen Krebsseren Antikörper gefunden wurden, die an Gangliosidstrukturen und an Gangliosidstrukturen simulierende Antigenstrukturen binden, und zwar spezieller an Asialo-G_{M1} bindende Antikörper (anti-AG_{M1}-Antikörper) und/oder damit kreuzreagierende, an Monosialo-G_{M1} bindende Antikörper (anti-G_{M1}-Antikörpern) vom IgG- und/oder IgA-Typ. Die Anwesenheit von Antikörpern, die an Asialo-G_{M1} binden und damit NK-Zellen inhibierende Eigenschaften aufweisen, und/oder ihre gegenüber Normalpersonen signifikant erhöhe Menge sind somit direkt mit dem Vorliegen eines Neoplasmas und/oder einer erhöhten Gefährdung des Patienten durch maligne Neoplasmen korrelierbar.

Ein Auftreten von NK-Zellen inhibierenden Antikörpern läßt sich ursächlich mit der Entstehung und Ausbreitung eines Krebsgeschehens in Verbindung bringen, woraus sich einem Patentschutz zugängliche Verwendungen und Mittel ableiten lassen, die an der Inhibierung/Eliminierung derartiger Antikörper ansetzen und für die in den Patentansprüchen 1 bis 8 Schutz begehrt wird.

Die Bedeutung der diagnostischen Bestimmung von anti-Gangliosid-Antikörpern bzw. -Autoantikörpern für die Diagnose und für die Therapie der Krebsentstehung bzw. -entwicklung wird im zweiten Teil dieser Anmeldung ausführlicher erläutert.

Die vorliegende Erfindung ist ein unerwartetes Ergebnis der intensiven Forschungen der Anmelderin auf dem Gebiet der klinischen Diagnose von Autoimmunerkrankungen. Sie gingen aus von der bekannten Erkenntnis, dass zu den Antikörpern, die in der Literatur im Zusammenhang mit Autoimmunerkrankungen, insbesondere nervenschädigenden, neuropathischen Autoimmunerkrankungen, diskutiert werden, auch bestimmte Anti-Gangliosid-Antikörper gehören.

Ganglioside sind Glykolipide, die Bestandteile der extrazellulären Seite der Plasmamembran tierischer Zellen sind und als solche auch im Nervengewebe auftreten. Sie enthalten pro Mol mehrere Monosaccharid-Einheiten, jedoch keinen PhosphorGehalt und werden den Sphingolipiden zugeordnet. Verglichen mit Proteinen sind sie eher niedermolekulare Biomoleküle. Die Ganglioside, an die die im Rahmen der vorliegenden Erfindung diskutierten Antikörper binden, sind das allgemein als G_{M1} bezeichnete Monosialo-Gangliosid bzw. insbesondere die zugehörige "Asialo"-verbindung AG_{M1}. G_{M1} weist eine Polysaccharidkette aus 4 Zucker-Monomereinheiten auf, die zwei D-Galactoseein-heiten, eine N-Acetylgalactosamin- und eine D-Glucoseeinheit umfassen, wobei letztere an einen Ceramidteil gebunden ist. An die innerhalb der Polysaccharidkette angeordnete D-Galactoseeinheit ist bei dem Gangliosid G_{M1} ein N-Acetylneuraminsäurerest (NANA; Sialsäure- oder o-Sialinsäurerest; "Monosialo"-Rest) gebunden, der bei dem sialinsäurefreien Asialo-G_{M1} (AG_{M1}) fehlt.

Die genannten Ganglioside und verwandte Verbindungen werden mit zahlreichen wichtigen biologischen Funktionen des menschlichen Körpers in Zusammenhang gebracht, zu denen z.B. das exonale Wachstum und die neuronale Diffenzierung, Rezeptorenfunktionen und Beteiligungen an verschiedenen Immunreaktionen des Körpers sowie an der Signaltransduktion und Zell-Zell-Erkennung gehören.

Es ist seit langem bekannt, dass im menschlichen Körper Antikörper bzw. Autoantikörper auftreten können, die an das Gangliosid G_{M1} binden, insbesondere dessen Kohlenhydratstrukturen, und an diesen ähnelnde ("diese simulierende") Kohlenhydratstrukturen anderer Moleküle. Die physiologische Rolle solcher Antikörper sowie ihre eventuelle Bedeutung für die klinische Diagnostik ist Gegenstand zahlreicher wissenschaftlicher Untersuchungen. Eine Korrelation der Befunde für anti-G_{M1}-Antikörper mit an sich bekannten Eigenschaften von an Asialo-G_{M1} bindenden Antikörpern wurde in diesem Zusammenhang noch nicht hergestellt.

Der weitaus überwiegende Teil aller veröffentlichten Arbeiten befaßt sich mit der Rolle und der diagnostischen Signifikanz von Anti-Gangliosid-Antikörpern bei Neuropathien, z.B. bei immunvermittelten motorischen Neuropathien wie dem Guillain-Barré-Syndrom (Radikuloneuritis, Polyradikulitis) und dem verwandten (Miller-)Fisher-Syndrom. Auch im Zusammenhang mit der Alzheimer-Erkrankung wurde bereits von einem vermehrten Auftreten von Anti-G_{M1}-Autoantikörpern bei einigen Patienten berichtet. Ferner wurden sie bei einzelnen HIV-Patienten gefunden. Von einzelnen Versuchen ihrer Bestimmung im Zusammenhang mit bestimmten Krebsarten wurden ebenfalls berichtet, wobei jedoch nur wenig aussagekräftige Ergebnisse bzw. Ergebnisse mit einer niedrigen Sensitivität erhalten wurden. Es wird diesbezüglich auf die Diskussion weiter unten verwiesen.

Zur Vermeidung von ungerechtfertigt engen und einschränkenden Auslegungen der in der vorliegenden Anmeldung und den zugehörigen Ansprüchen verwendeten Begriffe sollen nachfolgend einige der wichtigsten Begriffe für die Zwecke der vorliegenden Anmeldung besonders definiert werden:
- "Antikörper":: Dieser Begriff umfaßt, ohne zwischen verschiedenen Entstehungs- und Bildungsarten zu unterscheiden, Antikörper sowohl gegen externe Antigene als auch gegen körpereigene Strukturen, d.h. Autoantikörper, wobei letztere ggf. auch durch Antigen-Kreuzreaktionen aus Antikörpern gegen externe Antigene zu Autoantikörpern geworden sein können und ihre Bindungsfähigkeit gegen externe Antigene bewahrt haben können.
Wenn z.B. davon gesprochen wird, dass ein Antikörper "an Gangliosidstrukturen und an Gangliosidstrukturen simulierende Antigenstrukturen" bindet oder gegenüber "Gangliosiden bzw. bestimmten Gangliosiden reaktiv" ist, wobei reaktiv "im Sinne einer spezifischen Bindung reaktiv" bedeutet, soll er durch diese Definition hinreichend definiert sein, ohne dass z.B. seine spezifische Bindung auch an zusätzliche andere antigene Strukturen, oder seine praktische Bestimmung unter Verwendung von Reagenzien (zur Immobilisierung bzw. Markierung bzw. als Kompetitoren) mit molekularen Strukturen, die AG_{M1}, insbesondere dessen Kohlenhydratstuktur, nur simulieren, für die Definition als erfindungsgemäßer Antikörper eine Rolle spielen sollen.
- "Gangliosid": Im Rahmen der vorliegenden Erfindung steht der Begriff "Gangliosid" bei der Kennzeichnung des Bindungsverhaltens der zu bestimmenden Antikörper in erster Linie für das Ganglioside AG_{M1}. Der Begriff soll jedoch auch bisher noch nicht untersuchte verwandte Ganglioside erfassen, z.B. fukosylierte Ganglioside, wenn sich noch zeigt, dass auch an diese Ganglioside bindende Antikörper mit einer vergleichbaren diagnostischen Signifikanz für Neoplasmen in Krebsseren gefunden werden.
- "Simulation": Wenn im Rahmen der vorliegenden Anmeldung davon gesprochen wird, dass außer den speziellen Gangliosiden (AG_{M1} und G_{M1}) auch deren Wirkung "simulierende" Substanzen bzw. Verbindungen eingesetzt werden können, ist damit gemeint, dass es Kohlenhydratstrukturen aufweisende Verbindungen gibt (zu denen auch baterielle Toxine gehören), die wie die genannten Ganglioside an die fraglichen Antikörper binden. Sie können daher potentiell wie die Ganglioside zur spezifischen Bindung der Antikörper (z.B. zu Zwecke ihrer Entfernung aus dem Blutkreislauf) oder zu ihrer Blockierung verwendet werden.
Im genannten Sinne das Bindungsverhalten von Gangliosiden "simulierende" Substanzen lassen sich z.B. mit Hilfe eines Screeningverfahrens erkennen, bei dem man eine biologische Probe (insbesondere ein Serum), die einen hohen Gangliosid-Antikörper-Titer aufweist und deren Bindungsverhalten in einem gegebenen Assay bestimmt wurde, mit einer zu testenden Kandidaten-Substanz in Kontakt bringt und die Antikörperbindung in dem gleichen Assay ein weiteres Mal bestimmt. Eine deutliche Erniedrigung oder Aufhebung der Antikörperbindung ist ein Zeichen dafür, dass die untersuchte Substanz eine "Ganglioside simulierende Substanz" ist. Dieser Test kann auch im Zusammenhang mit der Festellung einer Patentverletzung durch Mittel, die den in der vorliegenden Anmeldung offenbarten bzw. beanspruchten entsprechen, durchgeführt werden.

Weitere Begriffsbedeutungen ergeben sich für den Fachmann aus der einleitenden und nachfolgenden Beschreibung der Erfindung und ihrer Ausführungsbeispiele.

In der nachfolgenden Beschreibung wird, zusätzlich zu dem Inhalt einer Tabelle, auch auf Figuren Bezug genommen, die zeigen:
- Fig. 1: eine graphische Darstellung der Ergebnisse der Messung von Antikörpern der IgG-Klasse, die an Monosialo-G_{M1} binden, in Seren von 137 Kontroll-personen, gegenübergestellt den Ergebnissen der Vermessung von Seren von 147 Tumorpatienten;
- Fig. 2: die Ergebnisse einer Vermessung der gleichen Seren wie in Fig. 1 auf Antikörper der IgA-Klasse, die an Monosialo-G_{M1} binden;
- Fig. 3: ein Diagramm, in dem eine Aufschlüsselung der Antikörperbestimmung in den Tumorseren gemäß Fig. 1 bezüglich der klinisch diagnostizierten Tumorarten/Erkrankungen erfolgt, die durch Zahlen von 1 bis 14 gekennzeichnet sind, wobei die Bedeutung der Zahlen 1 bis 14 in der nachfolgenden Beschreibung erläutert wird;
- Fig. 4: eine der Aufschlüsselung von Fig. 3 entsprechende Aufschlüsselung der Messung von Antikörpern der IgA-Klasse gemäß Fig. 2;

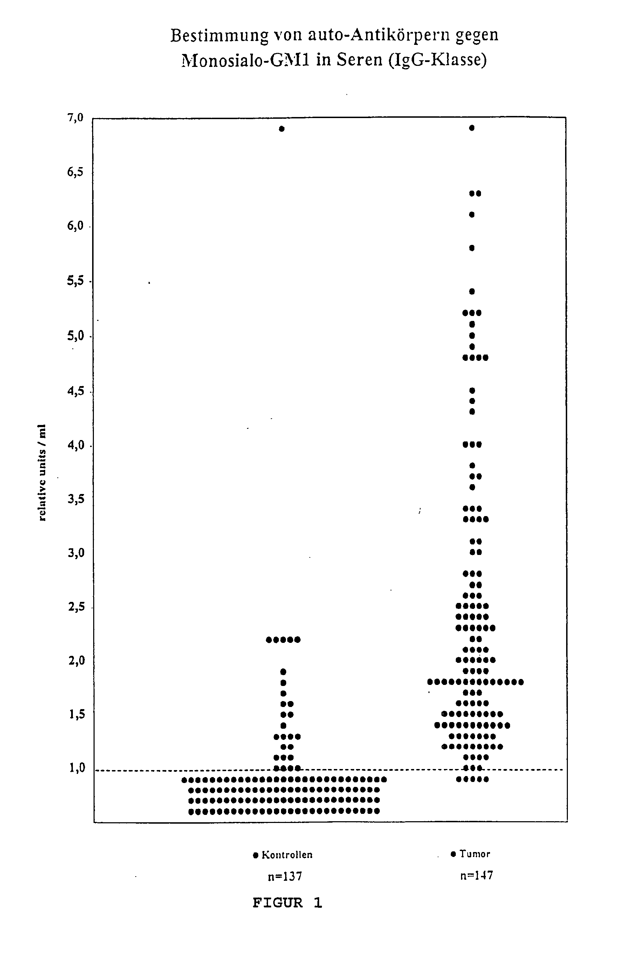

Für an Asialo-G_{M1} (AG_{M1}) bindende Antikörper, denen im Rahmen der vorliegenden Erfindung besondere Bedeutung zukommt, liegen den Figuren 1 bis 4 entsprechende Befunden für ein etwas kleineres Kollektiv von Seren vor. Es wird auf die entsprechenden Versuchsergebnisse in der gleichzeitig eingereichten weiteren Patentanmeldung der Anmelderin verwiesen.

Nachfolgend werden zuerst die überraschenden analytischen Befunde, die der vorliegenden Erfindung zugrunde liegen, beispielhaft erläutert. Eine ausführlichere Darstellung findet sich in einer gleichzeitig eingereichten weiteren Anmeldung der Anmelderin, auf die zur Ergänzung des Offenbarungsgehalts der vorliegenden Anmeldugn ausdrücklich Bezug genommen wird:

### 1. Vermessung von Seren von gesunden Normalpersonen (Kontrollen) und Krebspatienten auf an die Ganglioside G_{M1} und AG_{M1} bindende (Auto-)Antikörper

Unter Verwendung von mit Gangliosiden (G_{M1} und AG_{M1}) beschichteten Teströhrchen (GA-CTs), deren freie Bindungsstellen mit BSA abgesättigt worden waren, wurden Reihenmessungen an Kontroll- und Testseren vorgenommen. Dabei wurden, wie in der gleichzeitig eingereichten weiteren Anmeldung der Anmelderin, auf deren Inhalt zur Ergänzung des Inhalts der vorliegenden Anmeldung ausdrücklich Bezug genommen wird, näher beschrieben ist, in einem ersten Inkubationsschritt an die zur Beschichtung verwendeten Ganglioside bindende Antikörper aus dem jeweiligen Serum (Kontroll- und Testserum) gebunden, und dann wurden zur separaten Bestimmung von Antikörpern vom IgG- und vom IgA-Typ die gebundenen Antikörper mit markierten tierischen (Ziege) anti human-IgG- bzw anti human-IgA-Immunglobulinen nachgewiesen. Die gebundenen markierten Immunglobuline wurden anhand ihrer Markierung (Acridiniumester als Chemilumineszenz-Marker) detektiert bzw. quantifiziert. Zur Gewinnung eines für die Antikörpermenge repräsentativen Messsignals muss ein Hintergrundsignal in Abzug gebracht werden, das unter identischen Messbedingungen für das jeweiige identische Serum mit bis auf die fehlende Gangliosidbeschichtung identischen Teströhrchen gewonnen wurde.

Als Kontrollseren für die Antikörper-Bestimmungen unter Verwendung von GA-CTs, die mit G_{M1} beschichtet waren, dienten 137 Kontrollseren (Blutspenderseren und - zur Vermeidung von Lebensalter-bedingten Einflüssen auf die Antikörperkonzentrationen - Seren von Normalpersonen verschiedenen Alters aus Pflegeheimen und von Mitarbeitern der Anmelderin). Für die Antikörper-Bestimmungen unter Verwendung von GA-CTs, die mit AG_{M1} beschichtet waren, wurde ein Teilkollektiv dieser Seren vermessen, das nur 30 Seren umfaßte.

Als Testseren für die Antikörper-Bestimmungen unter Verwendung von GA-CTs, die mit G_{M1} beschichtet waren, dienten 147 Seren von Patienten mit klinisch diagnostizierten Tumoren verschiedener Organe/Gewebe sowie, zusätzlich, 20 Seren von Patienten mit chronisch entzündlichen Darmerkrankungen (Morbus Crohn, Colitis Ulcerosa). Zu jedem Testserum existierte eine genaue klinische Dokumentation, die eine Aufschlüsselung der in die Messung eingesetzten Seren von Krebspatienten nach der bei ihnen festgestellten Tumorart ermöglichte. Für die Antikörper-Bestimmungen unter Verwendung von GA-CTs, die mit AG_{M1} beschichtet waren, wurde ein Teilkollektiv dieser Seren vermessen, das nur 30 Seren von Krebspatienten umfaßte.

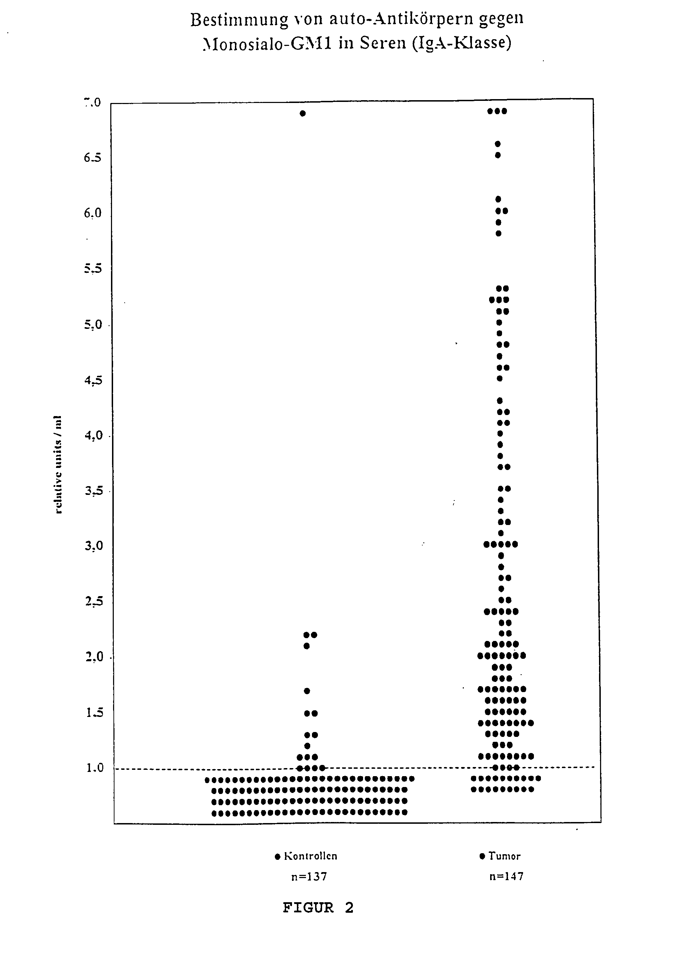

Die Ergebnisse der Bestimmungen von Antikörpern der Klassen IgG und IgA unter Verwendung von GA-CTs, die mit G_{M1} beschichtet waren, sind beispielhaft in den Figuren 1 und 2 gezeigt. Ausführlichere Messdaten finden sich wiederum in der parallelen Anmeldung der Anmelderin vom gleichen Tag.

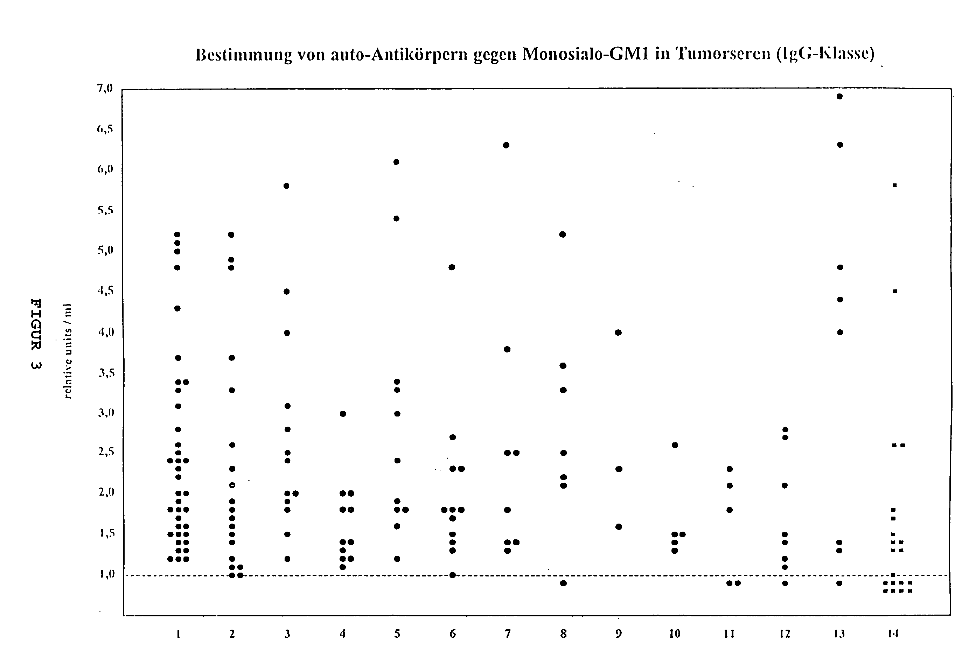

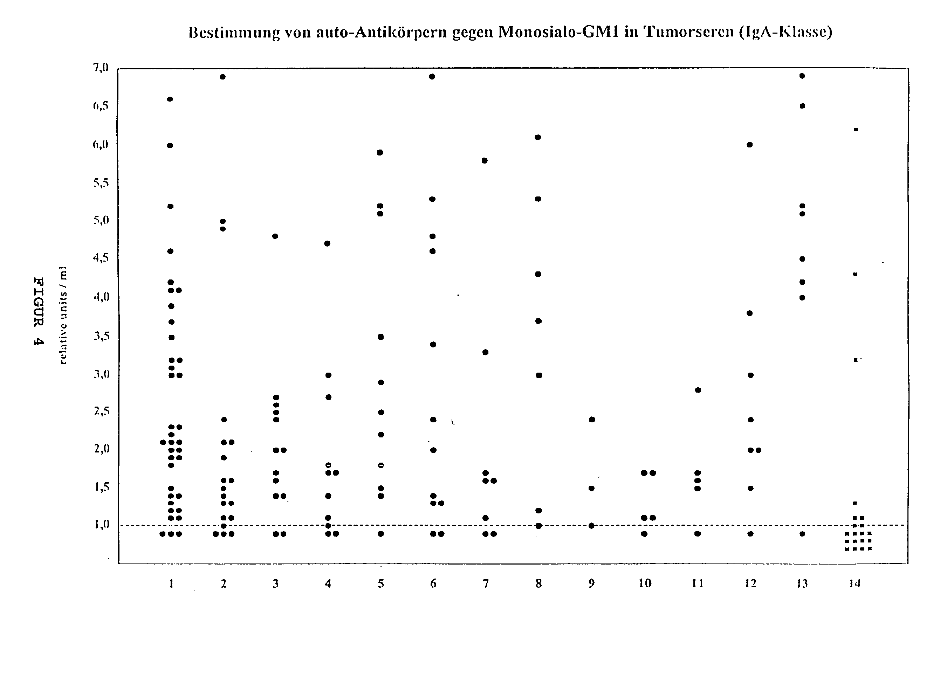

Die Figuren 3 und 4 zeigen eine Aufschlüsselung der Messergebnisse gemäß Figur 1 (IgG) bzw. Figur 2 (IgA) nach Krankheitsbildern. Dabei stehen die in waagerechter Richtung aufgetragenen Kennziffern für die der folgenden Aufstellung entnehmbaren klinischen Diagnosen:

| Kennziffer | Diagnose | Zahl der Seren |
|---|---|---|
| 1 | Colon-Karzinom | n = 37 |
| 2 | Mamma-Karzinom | n = 19 |
| 3 | Ovarial-Karzinom | n = 13 |
| 4 | Magen-Karzinom | n = 11 |
| 5 | Pankreas-Karzinom | n = 11 |
| 6 | Ösophagus-Karzinom | n = 12 |
| 7 | Gallen-Karzinom | n = 8 |
| 8 | Leber-Karzinom | n = 7 |
| 9 | C-Zell-Karzinom | n = 3 |
| 10 | Schilddrüsen-Karz. | n = 5 |
| 11 | Prostata-Karzinom | n = 5 |
| 12 | Lungen-Karzinom | n = 8 |
| 13 | Sonstige Karzinome | n = 8 |
| 14 | M.Crohn/C.Ulcerosa | n = 20 |

Die "Sonstigen Karzinome" teilten sich dabei wie folgt auf:
Appendix-Karzinom (n=1), Blasen-Karzinom (n=1), Cardia-Karzinom (n=1), Distales Verophagus-Karzinom (n=2), Mundboden (n=1), Nieren-Karzinom (n=1), Oberbauchtumor (n=1). Die nachfolgende Tabelle 1 faßt die Ergebnisse der Figuren 3 und 4 zahlenmäßig zusammen.

**Tabelle 1**

| Betroffenes Organ | anti G_{M1} IgG | anti GM1 IgA | n |
|---|---|---|---|
| Colon/Rektum | 38 = 100% | 35 = 92% | 38 |
| Mamma | 19 = 100% | 16 = 84% | 19 |
| Ovarien | 13 = 100% | 11 = 85% | 13 |
| Magen | 11 = 100% | 9 = 82% | 11 |
| Pankreas | 11 = 100% | 10 = 91% | 11 |
| Ösophagus | 12 = 100% | 10 = 83% | 12 |
| Galle | 8 = 100% | 6 = 75% | 8 |
| Leber | 6 = 86% | 7 = 100% | 7 |
| C-Zell | 3 = 100% | 3 = 100% | 3 |
| Lunge/ Bronchien | 7 = 88% | 7 = 88% | 8 |
| Schilddrüse | 5 = 100% | 4 = 80% | 5 |
| Prostata | 3 = 60% | 4 = 80% | 5 |
| sonstige | 7 = 88% | 7 = 88% | 8 |

Führt man zusätzlich eine quantitative Korrelation der einerseits mit G_{M1}-beschichteten Teströhrchen und andererseits, für das gleiche Seren-Teilkollektiv, mit AG_{M1}-beschichteten Teströhrchen erhaltenen Messergebnisse (in dieser Anmeldung nicht detailliert angegeben) für die IgG-Bestimmung durch, so ergibt sich eine weitgehende Übereinstimmung, die den Schluss erlaubt, dass bei beiden Bestimmungen wenigstens weitestgehend identische bzw. kreuzreagierende Antikörper gemessen wurden, d.h. Antikörper, die die Reaktivität von anti-AG_{M1}-Antikörpern aufweisen.

### 2. Diskussion der Befunde der Bestimmung von anti-Gangliosid-Antikörpern in Kontrollseren und in Seren von Krebspatienten

Wie die in den Figuren 1 bis 4 und der obigen Tabellen 1 beispielhaft zusammengefassten Messergebnisse eindrücklich zeigen, ermöglicht die Bestimmung von Antikörpern, die an Ganglioside (AG_{M1} und/oder G_{M1}) binden, ein klare Unterscheidung von Kontrollseren und Seren von Krebspatienten. Die Sensitivität der durchgeführten Bestimmungen lag für alle einzelnen Tumorarten über 75%, in den meisten Fällen sogar bei 100%.

Es ist ergänzend darauf hinzuweisen, dass bei einem Versuch, entsprechend den Bestimmungen von Antikörpern vom IgG- und IgA-Typ auch solche vom IgM-Typ zu bestimmen, keine diagnostisch relevanten Ergebnisse erhalten wurden (Ergebnisse nicht gezeigt).

Die gemessenen hohen Sensitivitäten in Verbindung mit einer hohen Unspezifität bezüglich der verschiedenen Krebsarten machen die Bestimmung von anti-Gangliosid-Antikörpern, insbesondere von solchen der IgG- und/oder IgA-Klassen, einerseits zu einem vielversprechenden Bestimmungsverfahren für die Diagnose, insbesondere für die Früherkennung, von Neoplasmen. Die bei allen Krebsseren, und zwar Seren von Patienten mit den verschiedensten Krebserkrankungen, signifikant erhöhten Titer von Antikörpern, die an AG_{M1} bzw. G_{M1} binden, deutet darauf hin, dass diese nicht im Sinne eines Epiphänomens erhöht sind, sondern höchstwahrscheinlich eine wichtige Rolle im Rahmen der Etablierung von malignen Neoplasmen (Tumoren) spielen, was sie zu therapeutischen Targets von hohem medizinischem Interesse macht:

Der wissenschaftlichen Literatur lassen sich Erkenntnisse, die auf eine wichtige Rolle von an Ganglioside bindenden Antikörpern (insbesondere an AG_{M1} und damit kreuzreagierend an G_{M1}) im Rahmen der Krebsentstehung verweisen, nicht entnehmen. Es wurden nur einige wenige Arbeiten bekannt, in denen der Versuch unternommen wurde, im Zusammenhang mit Krebserkrankungen auch anti-Gangliosid-Antikörper zu bestimmen. Ausgehend von dem Befund, dass beim sogenannten "small cell lung cancer" (SCLC) im Lungengewebe von Erkrankten eine erhöhte/abnormale Expression von Gangliosiden gefunden wurde, wurde in zwei Arbeiten (Grazyna Adler et al., Small cell lung cancer is not associated with the presence of anti-fucosyl-GM1 ganglioside autoantibodies reactive in immunoenzymatic test, Lung Cancer 34 (2001) 383-385; Aleksandra Lewartowska et al., Ganglioside reactive antibodies of IgG and IgM class in sera of patients with differentiated thyroid cancer, Immunol.Lett. 80 (2002) 129-132) untersucht, ob diese abnormale Gangliosidexpression dazu führt, dass bei Krebspatienten anti-Gangliosid-Antikörper gebildet und nachgewiesen werden können. In der erstgenannten Arbeit wurden die vorrangig gesuchten anti-Fukosyl-GM1-Gangliosid-Antikörper nicht gefunden, während in der zweiten der Arbeiten für den Fall von differenziertem Schilddrüsenkrebs (DTC) zwar in einer gewissen Anzahl von Patienten geringe Mengen an verschiedenen anti-Gangliosid-Antikörpern nachgewiesen werden konnten, jedoch z.B. im Falle der anti-GM1-Antikörper-Bestimmung die gefundenen Werte für die Krebspatienten unter denen der Kontrollen lagen (Fig.1). Nur im Hinblick auf eine Bindung an das fukosylierte Gangliosid FucGM1 wurden etwas stärker erhöhte Antikörpertiter gefunden. Im Lichte der Befunde der Anmelderin, die denen in den obigen Arbeiten klar widersprechen, ist anzunehmen, dass es den Autoren angesichts der erheblichen praktischen Schwierigkeiten bei der Bestimmung von anti-Gangliosid-Antikörpern vor einem intensiven, serumabhängigen Hintergrundsignal mit dem von ihnen verwendeten ELISA-Assay nicht gelang, tatsächlich aussagekräftige Messergebnisse zu produzieren. Eine mögliche pathogene Rolle der Antikörper, die bestimmt werden sollten, wird nicht suggeriert.

In einer Arbeit von Manousos M. Konstandoulakis et al., Autoantibodies in the Serum of Patients with Gastric Cancer: Their Prognostic Importance; Hybridoma, Vol.17, No.5, 1998, 431-435; wird - ähnliche Arbeiten der gleichen Autorengruppe weiterführend, im Rahmen derer an sich bekannte Autoantikörper bei Krebspatienten bestimmt wurden - ferner von einer Bestimmung von verschiedenen Typen von Antikörpern in Seren von Magenkrebspatienten berichtet, und es wird eine eventuelle prognostische Rolle einer solchen Bestimmung diskutiert. "Prognostisch" wird dabei im Sinne einer Prognose für den weiteren klinischen Verlauf einer bereits bestehenden und erkannten Krebserkrankung verwendet. Zur Antikörper-Bestimmung wird ein ELISA-Assay verwendet. Zu den Antikörpern, die gegenüber Kontrollen erhöht gefunden wurden, gehören auch sog. anti-G_{M1}-Antikörper, die allerdings nur mit einer Sensitivität von etwa 35% bei den Erkrankten gefunden wurden (gegenübergestellt einem Wert von 5% für Normalpersonen). Im Lichte der angegebenen Arbeit und von anderen Arbeiten der gleichen Autoren sind anti-Gangliosid-Antikörper nur eine von zahlreichen Arten von im Zusammenhang mit Krebs untersuchten Autoantikörpern, und aus den berichteten Daten läßt sich keinerlei Hinweis auf eine mögliche wichtige Rolle derartiges Antikörper bei der Entstehung und Entwicklung von malignen Neoplasmen, und damit auch keinerlei vielversprechender therapeutischer Ansatz, ableiten. Ein Schluss, dass die bestimmten anti-G_{M1}-Antikörper mit an AG_{M1} bindenden Antikörpern kreuzreagieren könnten und daher Eigenschaften von solchen anti-AG_{M1}-Antikörpern aufweisen könnten, wurde in diesem Zusammenhang nicht gezogen.

Eine solche Perspektive ergab sich erstmals aus den von der Anmelderin unter Einsatz ihrer großen Erfahrung auf dem Assaygebiet erhaltenen, vor dem Hintergrund des Standes der Technik außerordentlich überraschenden Messdaten, nämlich dem im Serum aller Krebspatienten signifikant erhöhten Auftreten von an das Gangliosid AG_{M1} bindenden Antikörpern und, damit kreuzreagierend, auch an G_{M1} bindenden Antikörpern.

Die Anmelderin konnte aufgrund der neuartigen Befunde der von ihr durchgeführten Messungen den Schluss ziehen, dass die bei Krebspatienten gefundenen erhöhten Titer von anti-Gangliosid-(Auto-)Antikörpern als Titer von NK-Zellen inhibierenden Antikörpern anzusehen sind, wodurch diesen Titern eine ganz neue gravierende medizinische Bedeutung zukommt, die nichts mit den Überlegungen zu tun hat, die Ausgangspunkt für die oben diskutieren Arbeiten waren, und dass sich aus den gefundenen erhöhten Titern von anti-AG_{M1}-Antikörpern und damit kreuzreagierenden anti-G_{M1}-Antikörpern neue, vielversprechende terapeutisceh Ansätze ableiten lassen:

Es ist bekannt, dass "natürliche Killerzellen" (NK-Zellen; cytotoxisch aktive Lymphozyten) an ihrer Oberfläche Asialo-G_{M1}-Strukturen aufweisen, an die anti-AG_{M1}-Antikörper spezifisch binden können, und dass diese in der Folge durch ihre Bindung die NK-Zellen desaktivieren und zerstören. Aktive NK-Zellen spielen jedoch eine außerordentlich wichtige Rolle im Rahmen der Immunabwehr des Menschen, indem sie z.B. alle entarteten körpereigenen Zellen, z.B. Krebszellen, abtöten, die aufgrund ihrer Entartung die Fähigkeit verloren haben, die NK-Zellen zu hemmen, die mit Ihnen in Kontakt kommen. Eine Beeinträchtigung (Hemmung, Abtötung) der normalen NK-Zellen, die deren selektiv-cytotoxische Eigenschaften aufhebt, führt daher dazu, dass im Verlaufe der natürlichen Lebensvorgänge entartete Zellen, insbesondere Zellen vom Tumorzell-Typ, nicht mehr ordnungsgemäß eliminiert werden. Aufgrund der verbesserten Überlebenschancen derartiger entarteter potentieller Tumorzellen können sich diese bei einer gestörten Funktion der NK-Zellen im Körper festsetzen, ungestört teilen und zu einem eigentlichen Tumor entwickeln. Es ist darauf hinzuweisen, dass es auf dem Gebiet von Tierexperimenten, bei den mit Versuchstieren gearbeitet wird, bei denen ein künstlicher Krebs erzeugt wird, bereits üblich ist, durch Gabe von anti-AG_{M1}-Antikörpern in Verbindugn mit einem krebsauslösenden Karzinogen oder einem Tumorkeim die Immunabwehr des Versuchstiers auszuschalten, so dass sich der - im Tiermodell gewünschte - experimentelle Krebs entwickeln kann (vgl. z.B. Hugh F. Pross et al., Role of Natural Killer Cells in Cancer, Nat Immun 1993; 12:279-292; Lewis L. Lanier et al., Arousal and inhibition of human NK Cells, Immunological Reviews 1997, Vol. 155:145-154; Yoichi Fuji et al., IgG Antibodies to AsialoGM1 Are More Sensitive than IgM Antibodies to Kill *in vivo* Natural Killer Cells and Prematured Cytotoxic T Lymphocytes of Mouse Spleen, Microbiol.Immunol. Vol. 34(6), 533-542, 1990; Carmine M. Volpe et al., AsGM1 + NK Cells Prevent Metastasis of Invading LD-MCA-38 Tumor Cells in the Nude Mouse, J Surg Res 84, 157-161 (1999); Susan D. Wilson et al., Correlation of Suppressed Natural Killer Cell Activity with Altered Host Resistance Models in B6C3F1 Mice, Toxicology and Applied Pharmacology 177, 208-218 (2001); H.Yoshino et al., Natural killer cell depletion by anti-asialo GM1 antiserum treatment enhances human hematopoietic stem cell engraftment in NOD/Shi-*scid* mice; Bone Marrow Transplantation (2000) 26, 1211-1216; N.Saijo et al., Analysis of Metastatic Spread and Growth of Tumor Cells in Mice with Depressed Natural Killer Activity by Anti-asialo GM1 Antibody or Anticancer Agents, J Cancer Res Clin Oncol (1984) 107: 157-163; Sonoku HABU et al., Role of Natural Kiler Cells against Tumor growth in Nude Mice - A Brief Review, Tokai J Exp Clin Med., Vol.8, No.5, 6: 465-468, 1983; Lewis L. Lanier, NK Cell Receptors, Annu. Rev. Immunol. 1998, 16: 359-93; Theresa L. Whiteside et al., The role of natural killer celss in immune surveillance of cancer; Current Opinion in Immunology 1995, 7:704-710; Tuomo Timonen et al., Natural killer cell-target cell interactions, Current Opinion in Cell Biology 1997, 9:667-673).

Ist bei einem menschlichen Individuum, z.B. aufgrund einer bakteriellen Exposition (z.B. Infektionen mit *Campylobacter jejuni* oder auch *Hellcobacter pylori;* vgl. 44 bis 54), die Produktion von anti-G_{M1}-Antikörpern einmal initiiert bzw. stark erhöht, entsteht unter Berücksichtigung der von der Anmelderin nachgewiesenen Kreuzreaktivität solcher Antikörper mit AG_{M1}-Epitopen (molekulare Mimikry) eine Voraussetzung für eine potentielle Schädigung von NK-Zellen und damit der Immunabwehr des Individuums - mit der Folge eines erhöhten Risikos, dass sich entartete Tumorzellen zu einem Tumor entwickeln können. Es ist also davon auszugehen, dass die in allen Krebsseren, die im Rahmen der o.g. Bestimmungen vermessen wurden, signifikant erhöht gefundenen anti-AG_{M1}-Antikörpertiter weniger im Sinne der bekannten Tumormarker eine Folge des Vorliegens eines Neoplasmas (Tumors) sind, sondern eher eine Voraussetzung bzw. ein fördernder Begleitumstand seines Entstehens. Im Laufe der Tumorentwicklung kann es dann, z.B. in Verbindung mit einer stimulierten NK-Zell-Aktivität, u.U. zur Umwandlung derartiger Antikörper in echte Autoantikörper und zu einem "Aufschaukeln" der Antikörpertiter kommen.

Da die mit Gangliosiden kreuzreagierenden Antikörper und die für deren Produktion erforderliche Prägung des Immunsystems bereits vor der Entwicklung eines Tumors vorhanden sein können, kann die Bestimmung von anti-AG_{M1}-Antikörpern zum Zwecke der Feststellung einer Disposition für die Entwicklung einer Krebserkrankung durchgeführt werden, und bei einem positiven Nachweis von Antikörpern, die an AG_{M1} und/oder G_{M1} binden, kann im Sinne einer Krebsprävention und/oder Krebshemmung bzw. Therapie interveniert werden.

Es ist in diesem Zusammenhang ferner von hohem Interesse, dass, wie insbesondere den Figuren 3 und 4 zu entnehmen ist, erhöhte Titer von mit anti-AG_{M1}-Antikörpern kreuzreagierenden anti-G_{M1}-Antikörpern auch bei einem Teil von Patienten gefunden werden, die an einer chronisch entzündlichen Darmerkrankung (Morbus Crohn, Colitis Ulcerosa) leiden. Es ist bekannt, dass z.B. bei Colitis Ulcerosa eine erhöhtes Risiko besteht, dass sich bei den Patienten im späteren Verlauf Darmkrebs entwickelt. Das Risiko beträgt etwa 40%. Der Befund, dass dieser prozentuale Wert in der Größenordnung der Werte liegt, die in Seren von Patienten mit chronisch entzündlichen Darmerkrankungen (Kennziffer 14) für erhöhte anti-G_{M1}-Titer gefunden werden, d.h. eine Parallität von statistischem Krebsrisiko und einem Auftreten von anti-(A)G_{M1}-Antikörpern in den Seren einschlägig Erkrankter erkennbar wird, ist ein weiterer die o.g. Annahmen stützender Befund.

Die sich aufgrund der geschilderten Befunde ergebenden Konsequenzen für neuartige Verfahren zur Prävention, Hemmung und Therapie von Krebs sind wie folgt:
1. Maßnahmen zum intrakorporalen Blockieren der pathogenen Antikörper dadurch, dass man dem Patienten Mittel (die selbstverständlich die zu fordernde Verträglichkeit aufweisen müssen) verabreicht, die geeignet sind, die an AG_{M1} bindenden Antikörper abzusättigen ("zu blockieren"). Die Ganglioside selbst sind derartige Mittel, und ihre gute Verträglichkeit für humane Patienten ist aus anderen Zusammenhängen, in denen sie bereits verabreicht werden (sie werden in hohen Mengen an Parkinson-Patienten verabreicht), bekannt.
2. Entfernen der Antikörper aus der Zirkulation des Patienten, z.B. auf dem Wege einer extrakorporalen Bindung an feste Affinitätsmaterialien ("Plasmapherese"). Für die Herstellung der als Binder eingesetzten Affinitätssäulen können auch hochaffine Kohlenhydratstrukturen genutzt werden, die die Antikörper mit höherer Affinität binden als die Ganglioside selbst.
3. Abschwächung der körpereigenen Produktion der speziellen Antikörper durch Verabreichung von Mitteln für die Blockierung antigenpräsentierender Zellen oder zur Erzeugung einer T-Zellanergie.

## Patentansprüche

1. Verwendung der Ganglioside Asialo-G_{M1} (AG_{M1}) und/oder G_{M1} sowie von Substanzen, die den Kohlenhydratteil dieser Ganglioside im Hinblick auf eine Bindung an anti-AG_{M1}-Antikörper und/oder anti-G_{M1}-Antikörper simulieren, zur Herstellung eines Mittels zur Bindung oder Blockierung von anti-AG_{M1}-Antikörpern und/oder anti-G_{M1}-Antikörpern, die an natürliche Killerzellen (NKC) binden, für die Prävention, Hemmung und Therapie von maligenen Krebserkrankungen.

2. Verwendung der Ganglioside AG_{M1} und/oder G_{M1} sowie von Substanzen, die den Kohlenhydratteil dieser Ganglioside im Hinblick auf eine Bindung an anti-AG_{M1}-Antikörper und/oder anti-G_{M1}-Antikörper simulieren, zur Herstellung von Mitteln zur Krebsprävention, Krebshemmung und Krebstherapie durch Blockierung antigenpräsentierender Zellen oder zur Erzeugung einer T-Zellanergie.

3. Verwendung der Ganglioside AG_{M1} und/oder G_{M1} sowie von Substanzen, die den Kohlenhydratteil dieser Ganglioside im Hinblick auf eine Bindung an anti-G_{M1}-Antikörper und/oder anti-AG_{M1}-Antikörper simulieren, zur Herstellung eines Affinitätsmaterials zur extrakorporalen Entfernung von anti-AG_{M1}-Antikörpern und/oder anti-G_{M1}-Antikörpern, die an natürliche Killerzellen binden, aus dem Blutkreislauf eines Patienten.

4. Verwendung nach einem der Ansprüche 1 bis 3, **dadurch gekennzeichnet, dass** die Substanzen, die den Kohlenhydratteil dieser Ganglioside im Hinblick auf eine Bindung an anti-AG_{M1}-Antikörper und/oder anti-G_{M1}-Antikörper simulieren, **dadurch gekennzeichnet sind, dass** sie mit Hilfe eines Screeningverfahrens zu erkennen sind, bei dem man ein Serum mit einem hohen Gangliosid-Antikörper-Titer mit einer zu prüfenden Substanz versetzt und danach das Bindungsverhalten der Antikörper aus der Probe an ihren spezifischen Bindungspartner bestimmt und ein gegenüber der substanzfreien Probe vermindertes Bindungsverhalten mit einer Gangliosidsimulation korreliert.

5. Verwendung nach Anspruch 2 zur Herstellung eines Mittels für die Blockierung antigenpräsentierender Zellen oder zur Erzeugung einer T-Zellanergie für die Verabreichung durch Injektion oder für die orale Verabreichung.

6. Verwendung nach einem der Ansprüche 1 bis 5, **dadurch gekennzeichnet, dass** die mit dem Mittel therapeutisch oder präventiv zu behandelnde Krebserkrankung ein Colon-Karzinom, ein Mamma-Karzinom, ein Ovarial-Karzinom, ein Magen-Karzinom, ein Pankreas-Karzinom, ein Ösophagus-Karzinom, ein Gallen-Karzinom, ein Leber-Karzinom, ein C-Zell-Karzinom, ein Schilddrüsen-Karzinom, ein Prostata-Karzinom, ein Lungen-Karzinom, ein Appendix-Karzinom, ein Blasen-Karzinom, ein Cardia-Karzinom, ein Karzinom des distalen Verophagus, ein Mundboden-Karzinom, ein Nieren-Karzinom oder ein Oberbauchkarzinom ist.

7. Verwendung nach einem der Ansprüche 1 bis 4, **dadurch gekennzeichnet, dass** der mit dem Mittel präventiv oder therapeutisch zu behandelnde Patient an einer entzündlichen Darmerkrankung leidet und aufgrund des Nachweises von signifikant erhöhten Mengen von an AG_{M1} und/oder G_{M1} bindenden Antikörpern ein erhöhtes Darmkrebs-Risiko aufweist.

8. Mittel zur Prävention und Behandlung von Krebserkrankugnen, das neben pharmazeutisch annehmbaren Trägern Wirkbestandteile enthält, die an AG_{M1}- und/oder G_{M1}-Antikörper auf eine Weise binden, dass die Bindung dieser Antikörper an natürliche Killerzellen (NKC) ganz oder teilweise verhindert wird.
